# NEW EUROPEAN PATENT SPECIFICATION

(11) **EP 3 320 942 B2**
(45) Date of publication and mention of the opposition decision: **08.10.2025**
(45) Mention of the grant of the patent: 22.03.2023
(21) Application number: 17205818.2
(22) Date of filing: 05.08.2008
(51) Int. Cl.: A61M 16/22, A61M 16/08, A62B 19/00, A62B 9/04

(54) **DISPOSABLE ABSORBER HAVING AN ADAPTER AND A LIP SEAL**
WEGWERFABSORBER MIT EINEM ADAPTER UND EINER LIPPENDICHTUNG
ABSORBEUR JETABLE DISPOSANT D'UN ADAPTATEUR ET D'UN JOINT À LÈVRE

(30) Priority: 24.10.2007 DE 102007050853
(43) Date of publication of application: 16.05.2018
(62) Divisional of application: 08161871.2
(73) Proprietor: Drägerwerk AG & Co. KGaA, 23558 Lübeck (DE)
(72) Inventor: KLEINSCHMIDT, Lothar, 23628 Krummesse (DE)
(74) Representative: Haseltine Lake Kempner LLP

(56) References cited:
- EP-A1- 1 748 232
- EP-A2- 1 230 943
- WO-A1-01/51841
- WO-A1-97/10020
- WO-A1-97/10020
- DE-B3- 102004 020 133
- DE-B3- 102007 025 809
- US-A1- 2005 235 994
- US-A1- 2006 231 092

## Description

The present disclosure relates to an adapter for adapting an absorber holder to a breathing system, having a first gas passage and a second gas passage which extend into the region of the connection between the absorber holder and the adapter, and having seals at the first gas passage and the second gas passage in the region of the connection between the absorber holder and the adapter. In anaesthetic apparatus and also in diving equipment, absorber holders filled with soda lime are used to bind the CO₂ (carbon dioxide) contained in the exhaled air.

Disposable absorbers are often used. The use of a disposable absorber produces the following advantages for the user: direct contact with the soda lime is avoided when the absorber holder is being handled and there is no nuisance caused by dust., There is also an improvement in the completeness with which the absorber filling is used and a cost saving is thus achieved, because a change of absorber is possible at any time, such as even during an operation for example, and the absorber can thus be used until the filling is completely exhausted. As an alternative, use is also made of refillable absorber holders. Refilling then preferably takes place through the bottom of the absorber holder or its cover or laterally through its circumferential surface.

The absorber holder needs to be quickly and easily replaceable without causing any interference with respiration. An example of an absorber of the kind specified in the opening paragraph can be seen from DE 197 29 739 A1.

An absorber holder is usually fitted to the existing absorber receptacle of a breathing system by means of an adapter and if required an intermediate plate. The interface at the absorber receptacle of the breathing system or the interface at the intermediate plate forms for example, together with the interface at the adapter, a bayonet connection. Once the two interfaces have been inserted one in the other and have then been turned relative to one another, the breathing system and the adapter are clamped together with a gas-tight seal. The absorber holder is preferably connected to the adapter by means of a pivotable receiving plate having an integral snap-action closure. For this purpose, the receiving plate has for example guide grooves to receive corresponding guide ribs on the absorber holder. If the receiving plate can be pivoted to a position in which it is inclined at about 30° to the horizontal, then the absorber holder automatically slides to its end position, which is defined by a recess milled in the receiving plate. By pivoting the absorber holder and the receiving plate towards the adapter, the snap-action closure is caused to close. It is particularly important in this case for there to be a gas-tight connection between the adapter and the absorber holder.

Known in this connection are gaskets which are hooked into the adapter and which are held in place on a cylindrical inner bush in the adapter by undercuts. The gaskets make a planar, butt seal at an end face against cylindrical stub tubes on the absorber holders, which are also referred to as sealing "seats". Unevennesses, contamination in the form of lime dust, or damage, at the end-faces of the sealing seats may cause leaks.

There are also internal gaskets on a spring-loaded body acting as a valve which, during a locking operation, makes the connection for gas between the absorber holder and the breathing system. By, for example, pressing with a finger on a specially marked point on the receiving plate, the absorber holder is unlocked and the connection for gas between the breathing system and the absorber holder is broken. At the same time, a bypass is created within the adapter by means of the spring-loaded valve body acting as a valve and the connection for gas to the surroundings is broken. In this position, respiration takes place without the absorber being in the circuit.

A further difficulty lies in the fact that high forces are required to lock the adapter and the absorber holder, because tolerances on the dimensions of the individual components have to be taken up by deforming the gaskets to the appropriate degree. This causes increased wear and, as a result, premature failure of the gaskets.

The present invention provides an absorber holder according to the appended clam 1. Optional features are recited in the dependent claims.

Described herein is an absorber holder and an adapter in which the connection system between the breathing system and the absorber holder allows a gas-tight connection to be made easily and in such a way that leaks are avoided at the seals of the connecting system.

The adapter for adapting an absorber holder to a breathing system may comprise:
- a first and a second gas passage which extend into the region of the connection between the absorber holder and the adapter,
- a valve means along the run of the gas passages which has a seal which either seals off the gas passages from the valve means in such a way that the absorber holder is cut off from the run of gas passages, or which seals off the gas passages from the valve means in such a way that the absorber holder is in the run between the first and second gas passages, and is characterised in that
- the seal has sealing surfaces which extend at an angle to the end-face of the absorber holder.

What is mean by "at an angle to the end-face of the absorber holder" in connection with the invention is at an angle of more than 10°, and preferably of more than 30°, and as a particular preference of more than 45°, to the end-face of the absorber holder.

The advantage of the adapter lies in essence in its sealing properties, which are improved because an inner and/or outer valve seat on the absorber holder not only bears against a seal at the end-face but because sealing surfaces are also formed by circumferential surface of the valve seat which is perpendicular to the end-face of the absorber holder, and a depressed surface which is obtained as an extension of the valve seat, take the form of sealing surfaces. The sealing surface thus comprises a plurality of portions of surface which are inclined to, or offset from, one another, which gives improved sealing properties.

The portions of surface may also be divided in such a way that a first portion of surface is the end-face of a valve seat and a further portion of surface is situated on the circumferential surface of the valve seat. It is a particular reference, for there to be provided on the circumferential surface a plurality of portions of surface which have individual sealing lips associated with them.

In an example the first gas passage takes the form of an inner gas passage and the second gas passage takes the form of an outer gas passage which is arranged to be concentric with the latter.

It has also proved advantageous for the sealing surface to comprise a sealing seat which extends at the end-face relative to the absorber holder, and a sealing lip which bears against the circumferential surface of the sealing seat.

The sealing seat on the absorber is in particular a valve seat belonging to the valve means. In this case, an annular inner valve seat has for example a mean diameter of 22 mm (millimetres) and inside and outside diameters of 20 and 24 mm respectively. An annular outer valve seat has for example a mean diameter of 42 mm and the inside and outside diameters are 40 and 44 mm respectively. Measured from the top face of the absorber holder, the height of the seat is preferably 5.5 mm. The depressed surface follows on in an annular form on the outside and is preferably situated 2.5 mm below the top face of the absorber holder, which means that the height of the seat from the depressed surface is 8 mm. The depressed surface has a inside diameter of 44 mm and an outside diameter of 60.5 mm, and the outer circumferential surface of the depressed surface is preferably inclined, in the region of the outside diameter, at 60° to the end-face of the absorber holder.

The sealing lips may bear against a circumferential surface of the sealing seat and/or against an end-face formed by a depressed surface and/or against the end-face of a sealing seat.

In principle, it is possible for different seals to be combined in the case of an adapter or an absorber holder. In this way, a plurality of lip seals for example may bear against an outer sealing seat and an inner sealing seat may be part of a conventional gasket and may form a sealing surface at an end-face.

If necessary, it is possible for seals having sealing lips made of elastomers not to be used if for example a spring-loaded body acting as a valve and a valve seat whose surface is of a suitable nature butt against one another and the conditions of pressure on the two sides of the seal do not put the seal under any great stress.

What also shows itself to be particularly advantageous is for the sealing seat to be of an annular form having rounded edges and for more than one lip seal to bear against the circumferential surface of a sealing seat. In particular, a depressed surface is formed as an extension, to produce an end-face relative to the absorber holder, to allow a plurality of lip seals to be fitted against the latter.

The object of the invention is achieved by an absorber holder for adaption to a breathing system described above.

The invention will be explained in which follows by reference to the accompanying drawings. In the drawings
Fig. 1 is a diagrammatic schematic view in longitudinal section of an absorber holder having a connected adapter, in the locked position,
Fig. 2 is a diagrammatic schematic view in longitudinal section of the absorber holder having an adapter Fig. 1, in the pivoted-away position,
Fig. 3 is a diagrammatic schematic plan view of the absorber holder shown in Fig. 1, showing the inner and outer valve seats,
Fig. 4 is a diagrammatic schematic view in cross-section of part of the absorber holder shown in Fig. 1, looking in the direction B indicated in Fig. 3,
Figs. 5 to 9 are diagrammatic views in profile of various sealing rings, and
Fig. 10 is a diagrammatic schematic view in cross-section of part of the absorber holder, showing a sealing ring in profile.

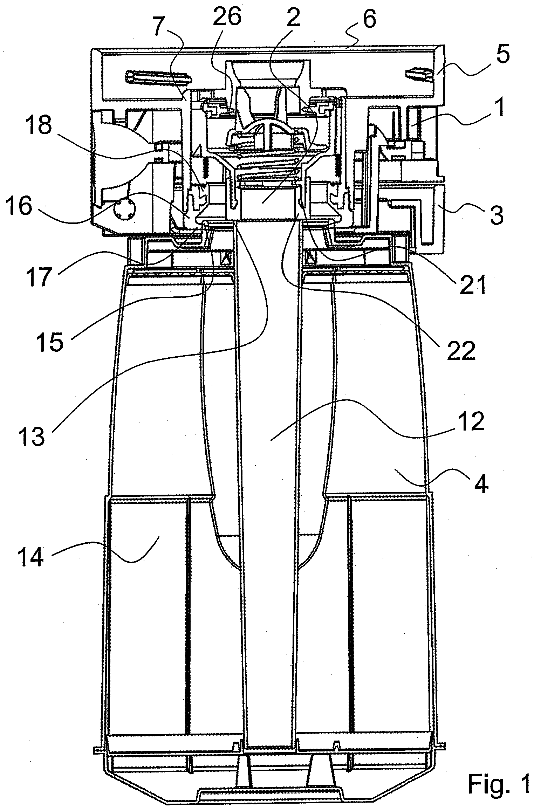

Fig. 1 is a schematic view in longitudinal section of an absorber holder 4 having a connected adapter 1, in the locked position. The adapter 1 has a main body 5 having a connecting cup 6 for connection to a breathing system which is not shown in Fig. 1. To connect the absorber holder 4 to the adapter 1, the absorber holder 4 is inserted in a receptacle 3 and pivoted towards the adapter 1, which has already been done in Fig. 1. The absorber holder 4 has an inner gas passage 12 having an inner valve seat 13 and an outer gas passage 14, arranged to be concentric with the inner gas passage 12, having an outer valve seat 15. The inner gas passage 12 defines the path of flow from the breathing system to the absorber holder 4 when the connected adapter 1 is in the locked position, and the outer gas passage 14 defines the path of flow from the absorber holder 4 back to the breathing system when the connected adapter 1 is in the locked position.

Within the adapter 1, the inner gas passage 12 extends through the interior of a valve means 2. Situated at the underside of a guiding sleeve 7 to receive the valve means 2, there is a first sealing ring 16 which has an outer sealing lip 17 directed towards the absorber holder 4 and an inner sealing lip 18. A portion 21 of the wall of the housing of the valve means 2 is provided with a second sealing ring 22 at its free end which extends towards the absorber holder 4.

When the absorber holder 4 is connected to the adapter 1, the outer sealing lip 17 of the first sealing ring 16 rests on the outer valve seat 15, and the second sealing ring 22 rests on the inner valve seat 13.

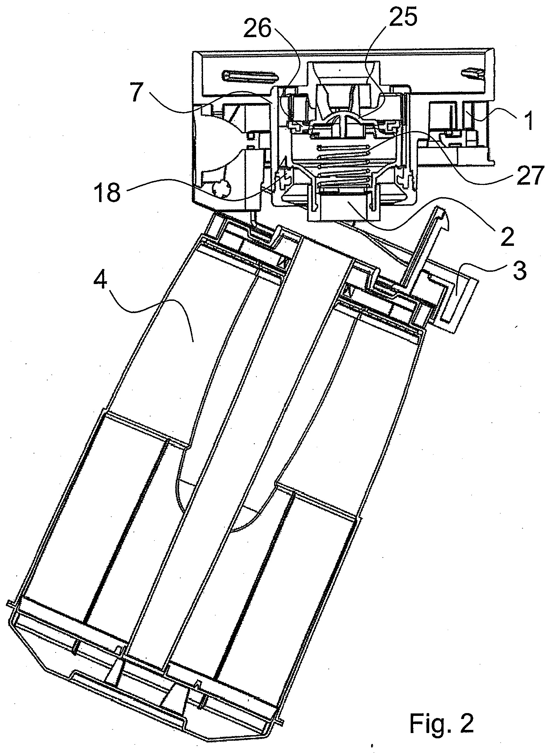

Fig. 2 is a schematic view in longitudinal section of the absorber holder 4 and adapter 1 of Fig. 1 in the pivoted-away position. The same reference numerals will be used in what follows for the same components as in Fig. 1.

Within the adapter 1, the path of flow extends through the interior of the valve means 2. The inner sealing lip 18 of the first sealing ring 16 bears from the outside against the housing of the valve means 2. The inner sealing lip 18 and the housing of the valve means 2 form a sealing region to stop the flow of gas from the absorber holder 4 when the absorber holder 4 is not connected to the adapter 1. A sealing lip 26 bears against a body 25 acting as a valve at the top end of the valve means 2. The sealing lip 26 and the body 25 acting as a valve, which latter pressed against the sealing lip 26 by a compression spring 27, form a further sealing region to stop the flow of gas to the absorber holder 4. When the absorber holder 4 is in the position shown in Fig. 2, the path for flow extends via the inner gas passage 12 and through openings within the adapter 1 to the outer passage 14, without following the path through the absorber holder 4.

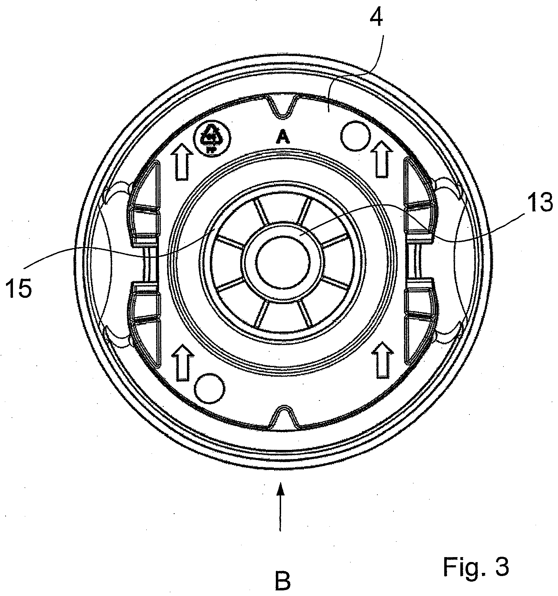

Fig. 3 is a schematic plan view of the absorber holder 4 showing the inner valve seat 13 and the outer valve seat 15.

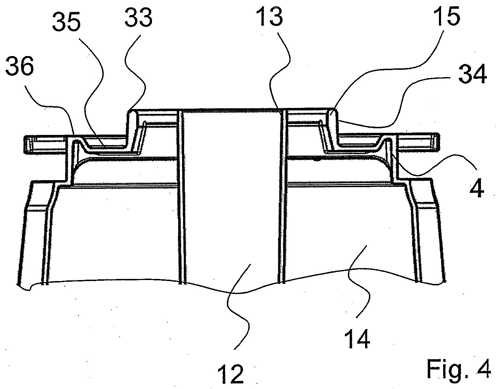

Fig. 4 is a schematic view in cross-section of part of the absorber holder 4, looking in the direction indicated by B in Fig. 3, showing the inner valve seat 13 and the outer valve seat 15. The outer valve seat 15 has an end-face 33 and a circumferential surface 34, from which latter a depressed surface 35 follows on in an annular form in the outward direction, on the top face 36 of the absorber holder 4. In the region of the outer circumference, the outer circumferential surface of the depressed surface 35 is inclined at 60° to the top face 36 of the absorber holder.

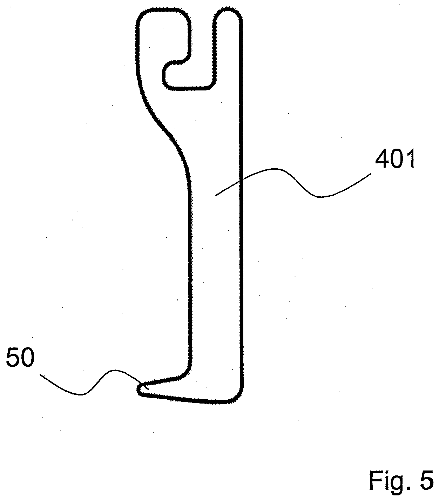

Figs. 5 to 9 are views in profile of various sealing rings 401, 402, 403, 404, 405 having sealing lips 50, 51, 52, 53, 54. The sealing rings 401, 402, 403, 404, 405 are first sealing rings 16. The sealing lips 50, 51, 52, 53, 54 are outer sealing lips 17.

Fig. 5 shows in profile a sealing ring 401 having a sealing lip 50 situated at the bottom.

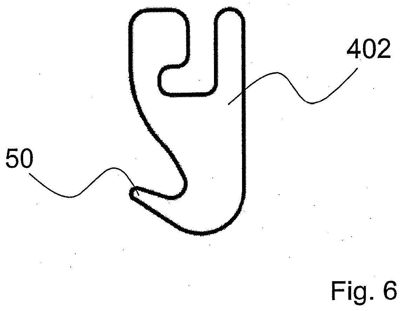

As an alternative, Fig. 6 shows in profile a sealing ring 402 having a sealing lip 50 situated towards the top.

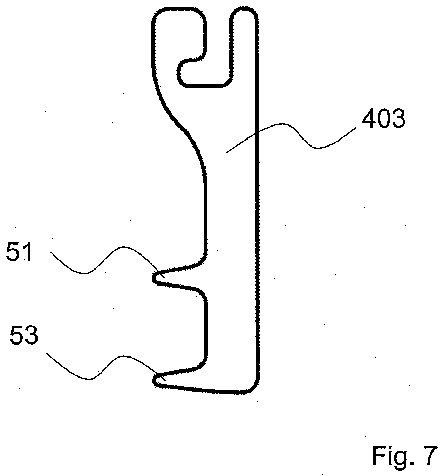

Fig. 7 shows in profile a further variant, which is a sealing ring 403 having an annular sealing lip 51 situated towards the top and an annular sealing lip 53 situated at the bottom.

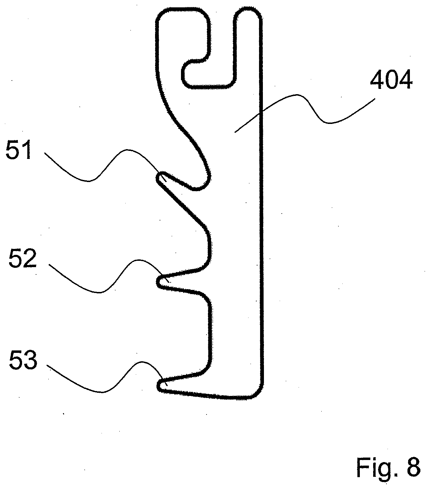

Fig. 8 shows in profile an additional variant, which is a sealing ring 404 having a sealing lip 51 situated towards the top, a sealing lip 52 situated in the centre and a sealing lip 53 situated at the bottom. The sealing lips 51, 52, 53 all bear against the circumferential surface 34 of the outer valve seat 15.

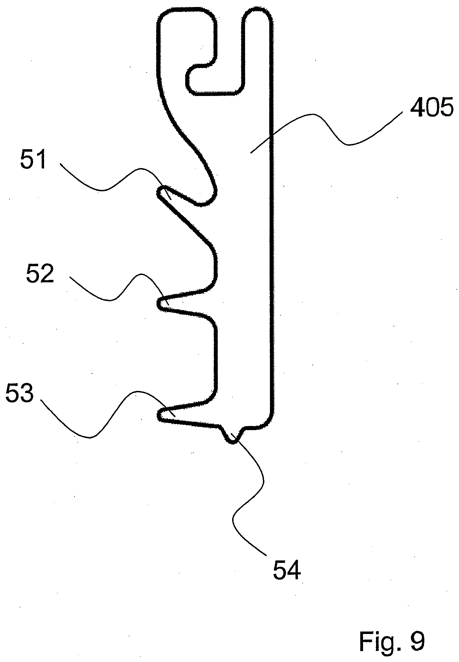

Fig. 9 shows in section a further variant, which is a sealing ring 405 having a sealing lip 51 situated towards the top, a sealing lip 52 situated in the centre, a sealing lip 53 situated at the bottom and a sealing lip 54 situated underneath, which sealing lips are each annular in form.

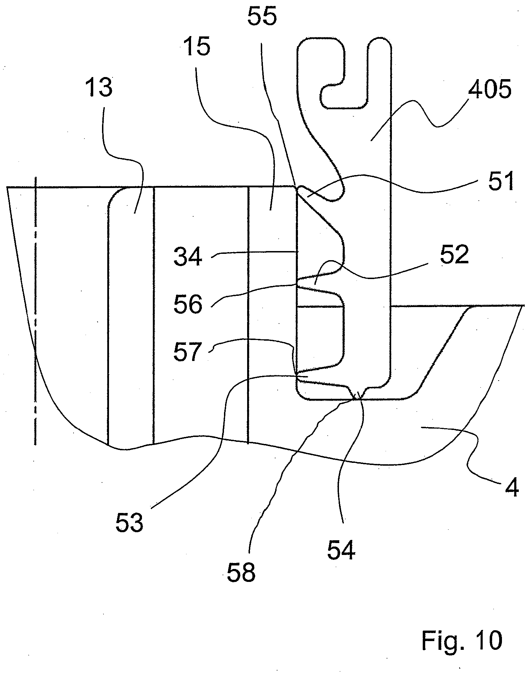

Fig. 10 shows the sealing ring 405 of Fig. 9 when co-operating with the outer valve seat 15 of the absorber holder 4. The sealing lips 51, 52, 53 bear in this case against portions 55, 56, 57 of the circumferential surface 34 of the outer valve seat 15. The underneath sealing lip 54 is situated against a further portion of surface 58 within the depressed surface 35.

## Claims

1. An absorber holder (4) for adaption to an adapter (1) of a breathing system, comprising:
an inner gas passage (12) having an inner valve seat (13); and
an outer gas passage (14) arranged to be concentric to the inner gas passage (12) and having an outer valve seat (15),
wherein one of the inner gas passage (12) and the outer gas passage (14) is configured to define a flow path from the breathing system to the absorber holder, and other of the inner gas passage (12) and the outer gas passage (14) is configured to define a flow path from the absorber holder back to the breathing system;
wherein each of the inner and outer valve seats (13,15) are configured to seal against a respective seal of the adapter,
wherein the inner and outer valve seats (13,15) each comprise a respective sealing end face (33, 35), and
wherein at least one of the inner and outer valve seats (13,15) comprises:
a circumferential sealing surface (34) configured for sealing against a respective seal of the adapter, the circumferential sealing surface extending substantially perpendicular to the end-face (33) of the absorber holder, wherein each of the circumferential sealing surface or surfaces (34) comprise a plurality of surface portions each configured to seal against a respective sealing lip of the respective seal of the adapter (4); and
wherein the absorber holder (4) further comprises a depressed surface (35) comprising a depressed end-face arranged annularly outwards of the outer valve seat (15), the depressed end-face being configured for sealing against a seal of the adapter.

2. An absorber holder (4) as claimed in claim 1, wherein the inner and outer valve seats (13,15) each comprise a respective circumferential sealing surface.

3. An absorber holder (4) as claimed in claim 1 or 2, wherein the circumferential sealing surface of the outer valve seat (15) is an outer circumferential surface.

4. An absorber holder (4) as claimed in any preceding claim, wherein a top face (36) of the absorber holder is arranged outward of the depressed surface (35), an outer circumferential surface of the depressed end-face being inclined to the top face of the absorber holder at an angle of 60 degrees

5. An absorber holder (4) as claimed in any preceding claim, wherein at least one of the inner and outer valve seats (13, 15) are of annular form.

6. An absorber holder (4) as claimed in claim 5, wherein at least one of the inner and outer valve seats (13, 15) comprise rounded edges.

7. An absorber holder (4) as claimed in any preceding claim, wherein the absorber holder comprises locking means for locking the absorber holder to an adapter.

## Patentansprüche

1. Absorberhalter (4) zur Anpassung an einen Adapter (1) eines Beatmungssystems, umfassend:
einen inneren Gasdurchgang (12) mit einer inneren Ventildichtung (13); und
einen äußeren Gasdurchgang (14), der so angeordnet ist, dass er konzentrisch zum inneren Gasdurchgang (12) ist, und einen äußeren Ventilsitz (15) aufweist,
wobei einer des inneren Gasdurchgangs (12) und des äußeren Gasdurchgangs (14) zum Definieren eines Strömungswegs vom Beatmungssystem zum Absorberhalter ausgelegt ist und der andere des inneren Gasdurchgangs (12) und des äußeren Gasdurchgangs (14) zum Definieren eines Strömungswegs vom Absorberhalter zurück zum Beatmungssystem ausgelegt ist;
wobei die inneren und äußeren Ventilsitze (13, 15) jeweils zum dichten Anliegen an einer jeweiligen Dichtung des Adapters ausgelegt sind,
wobei die inneren und äußeren Ventilsitze (13, 15) jeweils eine jeweilige abdichtende Endfläche (33, 35) umfassen und
wobei mindestens einer der inneren und äußeren Ventilsitze (13, 15) das Folgende umfasst:
eine umlaufende Dichtfläche (34), die zum dichten Anliegen an einer jeweiligen Dichtung des Adapters ausgelegt ist, wobei sich die umlaufende Dichtfläche im Wesentlichen senkrecht zu der Endfläche (33) des Absorberhalters erstreckt, wobei jede der umlaufenden Dichtfläche oder -flächen (34) eine Vielzahl von Oberflächenabschnitten umfasst bzw. umfassen, die jeweils zum dichten Anliegen an einer jeweiligen Dichtlippe der jeweiligen Dichtung des Adapters (4) ausgelegt sind, und
wobei der Absorberhalter (4) ferner eine vertiefte Oberfläche (35) umfasst, die eine vertiefte Endfläche umfasst, die vom äußeren Ventilsitz (15) ringförmig nach außen angeordnet ist, wobei die vertiefte Endfläche zum dichten Anliegen an einer Dichtung des Adapters ausgelegt ist.

2. Absorberhalter (4) nach Anspruch 1, wobei die inneren und äußeren Ventilsitze (13, 15) jeweils eine jeweilige umlaufende Dichtfläche umfassen.

3. Absorberhalter (4) nach Anspruch 1 oder 2, wobei die umlaufende Dichtfläche des äußeren Ventilsitzes (15) eine äußere Umfangsfläche ist.

4. Absorberhalter (4) nach einem der vorhergehenden Ansprüche, wobei eine Oberseite (36) des Absorberhalters von der vertieften Oberfläche (35) nach außen angeordnet ist, wobei eine äußere Umfangsfläche der vertieften Endfläche zur Oberseite des Absorberhalters in einem Winkel von 60 Grad geneigt ist.

5. Absorberhalter (4) nach einem der vorhergehenden Ansprüche, wobei mindestens einer der inneren und äußeren Ventilsitze (13, 15) ringförmig ist.

6. Absorberhalter (4) nach Anspruch 5, wobei mindestens einer der inneren und äußeren Ventilsitze (13, 15) abgerundete Ecken umfasst.

7. Absorberhalter (4) nach einem der vorhergehenden Ansprüche, wobei der Absorberhalter Verriegelungsmittel zum Verriegeln des Absorberhalters an einem Adapter umfasst.

## Revendications

1. Support d'absorbeur (4) destiné à être adapté sur un adaptateur (1) d'un système respiratoire, comprenant
un passage à gaz intérieur (12) comportant un siège de valve intérieur (13) ; et
un passage à gaz extérieur (14) disposé de façon à être concentrique par rapport au passage à gaz intérieur (12) et comportant un siège de valve extérieur (15),
l'un du passage à gaz intérieur (12) et du passage à gaz extérieur (14) étant conçu pour définir une voie d'écoulement du système respiratoire au support d'absorbeur, et l'autre du passage à gaz intérieur (12) et du passage à gaz extérieur (14) étant conçu pour définir une voie d'écoulement de retour du support d'absorbeur au système respiratoire ;
chacun des sièges de valve intérieur et extérieur (13, 15) étant conçu pour s'appliquer de manière étanche contre un joint d'étanchéité respectif de l'adaptateur, les sièges de valve intérieur et extérieur (13, 15) comprenant chacun une face d'extrémité d'étanchéité respective (33, 35), et
au moins un des sièges de valve intérieur et extérieur (13, 15) comprenant :
une surface d'étanchéité circonférentielle (34) conçue pour s'appliquer de manière étanche contre un joint d'étanchéité respectif de l'adaptateur, la surface d'étanchéité circonférentielle s'étendant sensiblement perpendiculairement à la face d'extrémité (33) du support d'absorbeur, la ou les surfaces d'étanchéité circonférentielles (34) comprenant chacune une pluralité de parties de surface conçues chacune pour s'appliquer de manière étanche contre une lèvre d'étanchéité respective du joint d'étanchéité respectif de l'adaptateur (4) ; et
le support d'absorbeur (4) comprenant, en outre, une surface en renfoncement (35) comprenant une face d'extrémité en renfoncement disposée de manière annulaire extérieurement vis-à-vis du siège de valve extérieur (15), la face d'extrémité en renfoncement étant conçue pour s'appliquer de manière étanche contre un joint d'étanchéité de l'adaptateur.

2. Support d'absorbeur (4) selon la revendication 1, dans lequel les sièges de valve intérieur et extérieur (13, 15) comprennent chacun une surface d'étanchéité circonférentielle respective.

3. Support d'absorbeur (4) selon la revendication 1 ou 2, dans lequel la surface d'étanchéité circonférentielle du siège de valve extérieur (15) est une surface circonférentielle extérieure.

4. Support d'absorbeur (4) selon l'une quelconque des revendications précédentes, dans lequel une face supérieure (36) du support d'absorbeur est disposée extérieurement vis-à-vis de la surface en renfoncement (35), une surface circonférentielle extérieure de la face d'extrémité en renfoncement étant inclinée par rapport à la face supérieure du support d'absorbeur à un angle de 60 degrés.

5. Support d'absorbeur (4) selon l'une quelconque des revendications précédentes, dans lequel au moins un des sièges de valve intérieur et extérieur (13, 15) est de forme annulaire.

6. Support d'absorbeur (4) selon la revendication 5, dans lequel au moins un des sièges de valve intérieur et extérieur (13, 15) comprend des bords arrondis.

7. Support d'absorbeur (4) selon l'une quelconque des revendications précédentes, le support d'absorbeur comprenant des moyens de solidarisation pour solidariser le support d'absorbeur avec un adaptateur.
